(19) Europäisches Patentamt
European Patent Office
Office européen des brevets

(11) **EP 2 376 882 B1**

(12) **FASCICULE DE BREVET EUROPEEN**

(45) Date de publication et mention de la délivrance du brevet:
**11.08.2021 Bulletin 2021/32**

(21) Numéro de dépôt: **10706703.5**

(22) Date de dépôt: **15.01.2010**

(51) Int Cl.:
*G01J 4/04* (2006.01)   *G01N 21/21* (2006.01)

(86) Numéro de dépôt international:
**PCT/FR2010/050063**

(87) Numéro de publication internationale:
**WO 2010/081999 (22.07.2010 Gazette 2010/29)**

(54) **DISPOSITIF ET PROCEDE DE DETERMINATION D'UNE INFORMATION DE POLARISATION ET IMAGEUR POLARIMETRIQUE.**

VORRICHTUNG UND VERFAHREN ZUR BESTIMMUNG VON POLARISATIONSINFORMATIONEN UND POLARIMETRISCHE BILDGEBUNGSVORRICHTUNG

DEVICE AND METHOD FOR DETERMINING A PIECE OF POLARISATION INFORMATION AND POLARIMETRIC IMAGING DEVICE

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **15.01.2009 FR 0950236**
**20.05.2009 FR 0953402**

(43) Date de publication de la demande:
**19.10.2011 Bulletin 2011/42**

(73) Titulaire: **Centre National de la Recherche Scientifique (C.N.R.S.)**
**75794 Paris Cedex 16 (FR)**

(72) Inventeurs:
 • **PAGNOUX, Dominique**
 **F-87100 Limoges (FR)**
 • **LOURADOUR, Frédéric**
 **F-87120 Eymoutiers (FR)**
 • **DESROCHES, Jérôme**
 **F-87100 Limoges (FR)**
 • **BARTHELEMY, Alain**
 **F-87100 Limoges (FR)**
 • **BREVIER, Julien**
 **F-87200 Saint-Junien (FR)**

(74) Mandataire: **Marks & Clerk France**
**Immeuble "Visium"**
**22, avenue Aristide Briand**
**94117 Arcueil Cedex (FR)**

(56) Documents cités:
EP-A2- 0 657 974    WO-A-98/53272
US-A- 4 626 679    US-B1- 6 292 287

• NIELSEN P M F ET AL: "POLARIZATION-SENSITIVE SCANNED FIBER CONFOCAL MICROSCOPE" OPTICAL ENGINEERING, SOC. OF PHOTO-OPTICAL INSTRUMENTATION ENGINEERS, BELLINGHAM, vol. 35, no. 11, 1 novembre 1996 (1996-11-01), pages 3084-3091, XP000638602 ISSN: 0091-3286
• MARTINELLI ET AL: "A universal compensator for polarization changes induced by birefringence on a retracing beam", OPTICS COMMUNICATIONS, ELSEVIER, AMSTERDAM, NL, vol. 72, no. 6 15 August 1989 (1989-08-15), pages 341-344, XP024466802, ISSN: 0030-4018, DOI: 10.1016/0030-4018(89)90436-7 [retrieved on 1989-08-15]

## Description

La présente invention concerne un dispositif et un procédé de détermination d'une information de polarisation en un point d'un échantillon cible, ainsi qu'un imageur polarimétrique.

En particulier, l'invention se rapporte à un dispositif de détermination du type comportant :

- une source lumineuse propre à émettre un faisceau lumineux polarisé rectilignement selon une direction prédéfinie, le faisceau lumineux étant destiné à être réfléchi par le point de mesure de l'échantillon cible ;
- des moyens de calcul de l'information de polarisation du point de mesure à partir du faisceau réfléchi par l'échantillon cible.

Le document US 7,289,211 décrit un exemple d'un tel dispositif de détermination.

Un dispositif de détermination d'informations de polarisation permet d'obtenir des informations sur la micro ou nanostructuration d'échantillons cibles, sur leur texture en surface ou à faible profondeur sous la surface. Ces informations de polarisation peuvent être, par exemple, le déphasage subi ou le degré de polarisation du faisceau renvoyé par l'échantillon cible. Ces informations sont principalement utilisées dans le domaine médical pour le diagnostic de maladies, et dans le domaine de la micro électronique pour caractériser des films minces monocouche, multicouches ou pour analyser des surfaces complexes.

Généralement, les informations de polarisation sont obtenues par réflexion d'un faisceau lumineux polarisé sur un échantillon cible. L'analyse de la polarisation du faisceau réfléchi permet de déterminer des informations de polarisation de l'échantillon cible.

Cette technique nécessite l'utilisation d'un faisceau lumineux en visée directe et en espace libre, de sorte qu'il n'est pas possible de réaliser des mesures d'informations de polarisation d'un objet situé dans une zone difficile d'accès, au sein de corps creux ou dans un environnement trouble. Le document "Polarization-sensitive scanned fiber confocal microscope", P. M. F. Nielsen et Al., Opt. Eng., 35(11), pp3084-3091, November 1996, décrit un dispositif similaire utilisant une fibre optique à maintien de polarisation combiné avec un retardateur variable et une lame quart d'onde à proximité de l'échantillon cible. Un imageur polarimétrique similaire est décrit dans US6292287 B1.

L'invention a notamment pour but de pallier l'utilisation d'éléments en espace libre ou d'éléments actifs près de l'échantillon cible et de proposer un dispositif de détermination d'une information de polarisation qui permette, entre autres, d'analyser des échantillons cibles non accessibles par un faisceau lumineux en visée directe.

A cet effet, l'invention a pour objet un dispositif de détermination du type précité, comme défini dans la revendication 1 et comportant :

- au moins un guide d'onde apte à guider le faisceau incident vers l'échantillon cible, et le faisceau réfléchi vers les moyens de calcul ; et
- des moyens de rotation de la polarisation propres à faire tourner deux composantes polarimétriques orthogonales $E_\parallel^I$ ; $E_\perp^I$ du faisceau incident après passage dans le guide d'onde et deux composantes polarimétriques orthogonales $E_\parallel^R$ ; $E_\perp^R$ du faisceau réfléchi avant passage dans le guide d'onde pour compenser l'effet de la biréfringence du guide d'onde.

Cette invention permet notamment d'analyser des structures de tissus biologiques telles que du collagène, *in vivo, in situ* et sans nécessité de biopsie.

L'invention a également pour objet un imageur polarimétrique propre à générer une image polarimétrique d'un échantillon cible, tel que défini dans la revendication 8, ledit imageur comportant :

- un dispositif de détermination d'une information de polarisation tel que décrit précédemment, ledit dispositif étant propre à déterminer plusieurs informations de polarisation ;
- une unité de construction d'une image polarimétrique représentative des informations de polarisation des points de mesure de l'échantillon cible, chaque caractéristique d'un pixel de l'image représentant l'information de polarisation d'un point de mesure de l'échantillon cible.

Enfin, l'invention a également pour objet un procédé de détermination d'une information de polarisation en un point de mesure d'un échantillon cible, tel que défini dans la revendication 11, ledit procédé comportant les étapes suivantes :

a) une étape d'émission d'un faisceau lumineux incident polarisé rectilignement selon une direction prédéfinie ;

b) une étape de guidage du faisceau incident vers le point de mesure de l'échantillon cible à l'aide d'un guide d'onde ;

c) une étape de rotation de deux composantes polarimétriques orthogonales du faisceau incident après passage dans le guide d'onde ;

d) une étape de réflexion du faisceau incident sur le point de mesure de l'échantillon cible ;

e) une étape de rotation de deux composantes polarimétriques orthogonales du faisceau réfléchi avant passage dans le guide d'onde ;

f) une étape de guidage du faisceau réfléchi vers une unité de calcul par le même guide d'onde ;

g) une étape de calcul de l'information de polarisation du point de mesure de l'échantillon cible à partir du faisceau réfléchi récupéré à la sortie du guide d'onde ; les étapes c) et e) compensent l'effet de la biréfringence du guide d'onde.

L'invention sera mieux comprise à la lecture de la description qui va suivre, donnée uniquement à titre d'exemple et faite en se référant aux dessins, sur lesquels :

- la figure 1 est une vue schématique du dispositif de détermination selon l'invention ;

- la figure 2 est un graphe représentant l'évolution temporelle de la composante polarimétrique perpendiculaire du champ électrique en fonction de la composante polarimétrique parallèle ;

- les figures 3-4 et 6 sont des vues schématiques d'un imageur polarimétrique selon quatre modes de réalisation de l'invention tandis que la figure 5 est une vue schématique d'un dispositif non couvert par les revendications; et

- la figure 7 est un diagramme des étapes du procédé selon l'invention.

Sur les différentes figures, les éléments désignés par la même référence désignent des éléments identiques ou similaires.

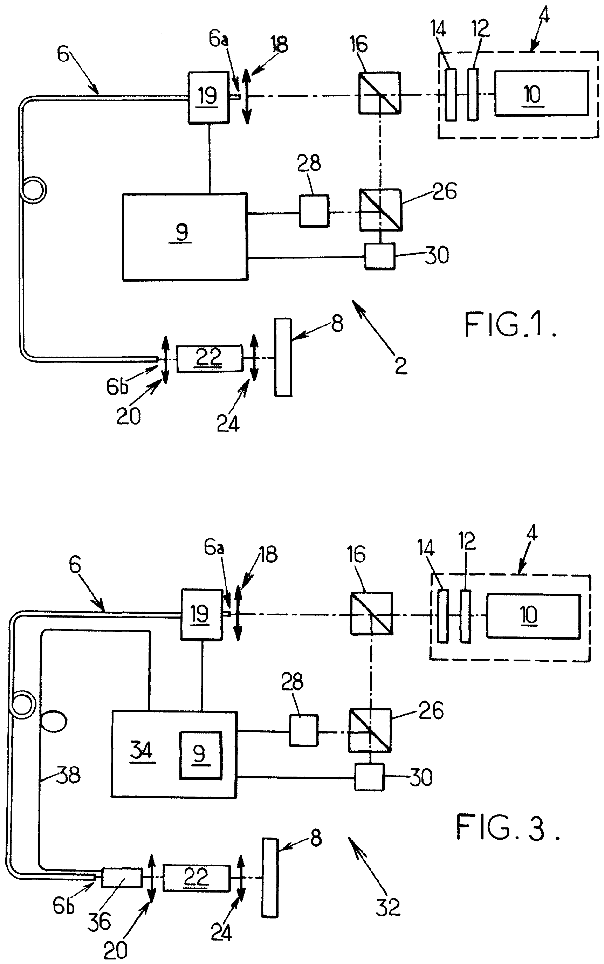

En référence à la figure 1, le dispositif de détermination 2 comporte une source lumineuse monochromatique 4 propre à émettre un faisceau lumineux incident, un guide d'onde 6 apte à être traversé par un faisceau incident et par un faisceau réfléchi par l'échantillon cible 8, et des moyens de calcul 9 de l'information de polarisation à partir du faisceau réfléchi récupéré à la sortie du guide d'onde 6.

Dans la suite de la description, le faisceau lumineux est appelé « faisceau incident » sur l'ensemble du trajet de la source 4 à l'échantillon cible 8, et « faisceau réfléchi » sur l'ensemble du trajet de l'échantillon cible 8 aux moyens de calcul 9.

Dans la suite de la description, les termes « amont » et « aval » sont définis en fonction du sens du faisceau lumineux.

La source lumineuse 4 est apte à émettre un faisceau lumineux incident polarisé rectilignement selon une direction prédéfinie x.

Cette source lumineuse 4 est, par exemple, constituée par une diode laser 10, un polariseur 12 et une lame demi-onde 14. Le polariseur 12 et la lame demi-onde 14 sont disposés en aval de la diode laser en considérant le sens du faisceau incident. Ils sont propres à être traversés par le faisceau émis par la diode laser 10. La source lumineuse 4 peut inclure un dispositif destiné à la protéger des réflexions externes.

Le dispositif de détermination 2 comporte entre la source lumineuse 4 et le guide d'onde 6, un cube 16 diviseur de faisceau ainsi qu'un système 18 de focalisation du faisceau incident dans le guide d'onde 6.

Le cube 16 est neutre à la polarisation. Il n'affecte que l'intensité du faisceau provenant de la source lumineuse 4 et se dirigeant vers le guide d'onde 6. Il est propre à modifier la direction du faisceau réfléchi pour le diriger vers les moyens de calcul 9.

Le système de focalisation 18 est constitué par exemple, par un objectif de microscope ou une lentille convergente dont le plan focal est situé à l'entrée du guide d'onde 6.

Le guide d'onde 6 est propre à guider le faisceau incident sur l'échantillon cible 8, en particulier lorsque ce dernier est positionné dans une cavité ou un renfoncement, ou même dans le corps humain, de sorte qu'il ne peut pas être atteint par transmission d'un faisceau lumineux en visée directe. Il comporte une extrémité 6a dite extrémité proximale, située à proximité de la source lumineuse 4 et des moyens de calcul 9, et une extrémité 6b dite extrémité distale, destinée à être disposée à proximité de l'échantillon cible 8.

Le guide d'onde 6 est constitué par une fibre optique monomode à la longueur d'onde du faisceau émis par la source lumineuse 4.

Des moyens 19 de variation de la biréfringence du guide d'onde 6 sont montés sur le guide d'onde 6. Ces moyens 19 ont pour effet de faire varier la polarisation du faisceau traversant le guide d'onde. Ces moyens de variation 19 comportent par exemple des moyens propres à faire vibrer le guide d'onde 6, des moyens aptes à générer un champ électrique ou des moyens aptes à faire varier la température ou la pression exercée sur celui-ci, afin de faire varier temporellement la polarisation du faisceau en sortie du guide d'onde 6.

Les moyens de variation 19 sont connectés aux moyens de calcul 9 et sont commandés par ceux-ci.

La traversée du guide d'onde 6 modifie le faisceau incident de sorte que, en sortie du guide d'onde 6, il présente deux composantes polarimétriques orthogonales $E_{\parallel}^{I}$ et $E_{\perp}^{I}$.

Cette modification est due à la fois à l'effet de la biréfringence du guide d'onde 6 et à l'effet des moyens de variation de la polarisation 19.

Entre l'extrémité distale 6b et l'échantillon cible 8, le dispositif de détermination 2 comporte en considérant le sens du faisceau incident, un premier système optique 20 dont le plan focal est situé au niveau de l'ouverture de l'extrémité distale du guide d'onde 6, des moyens de rotation de la polarisation 22, et un second système optique 24 dont le plan focal est situé au niveau de l'échantillon cible 8.

Le premier système optique 20 est propre à collimater le faisceau incident. Le second système optique 24 est propre à focaliser le faisceau incident sur le point de mesure de l'échantillon cible.

Après réflexion sur l'échantillon cible 8, le faisceau réfléchi est collimaté par le second système optique 24, il traverse les moyens de rotation 22 et, est focalisé par le premier système optique 20 à l'entrée de la partie distale 6b du guide d'onde.

Les moyens de rotation 22 comprennent un rotateur de Faraday. Pour les applications médicales, ce rotateur est miniaturisé.

Il permet de compenser les effets de la biréfringence du guide d'onde 6. A cet effet, il est propre à faire tourner les deux composantes polarimétriques orthogonales $E_\parallel^I$ et $E_\perp^I$ du faisceau incident sortant du guide d'onde 6, d'un angle de 45 degrés dans un sens de rotation donné pour obtenir deux composantes polarimétriques notées $E_\parallel^{IR}$ et $E_\perp^{IR}$. Dans cette description, le sens de la rotation est défini par rapport à un repère fixe de laboratoire.

Après réflexion du faisceau incident sur l'échantillon cible 8, le rotateur de Faraday est propre à faire tourner deux composantes polarimétriques orthogonales du faisceau réfléchi référencées $E_\parallel^R$ et $E_\perp^R$ d'un angle de 45 degrés dans le même sens de rotation pour obtenir deux composantes polarimétriques orthogonales référencées $E_\parallel^{RR}$ et $E_\perp^{RR}$.

Comme l'échantillon cible 8 a modifié la polarisation du faisceau incident, les composantes polarimétriques $E_\parallel^{IR}$ et $E_\perp^{IR}$ du faisceau incident en aval des moyens de rotation 22 sont différents des composantes polarimétriques $E_\parallel^R$ et $E_\perp^R$ du faisceau réfléchi en amont des moyens de rotation 24. Le terme aval est défini en considérant le sens du faisceau incident. Le terme amont est défini en considérant le sens du faisceau réfléchi.

Le guide d'onde 6 est propre à guider le faisceau réfléchi vers le système de focalisation 18.

Au cours de la seconde traversée du guide d'onde 6, les composantes polarimétriques $E_\parallel^{RR}$ et $E_\perp^{RR}$ issues du rotateur 22 sont à nouveau modifiées par le guide d'onde 6 et par les moyens de variation 19 de façon identique ou du moins similaire aux modifications occasionnées au cours du trajet vers l'échantillon cible 8.

Le dispositif de détermination 2 comprend en outre un cube 26 séparateur de polarisation et deux photodétecteurs 28, 30 reliés aux moyens de calcul 9.

Le cube 26 est conformé pour séparer une composante polarimétrique $E_\parallel^F$ orientée dans la direction prédéfinie x, c'est-à-dire dans la direction dans laquelle la source lumineuse 4 a polarisé le faisceau incident, et une composante polarimétrique $E_\perp^F$ orthogonale à celle-ci.

La composante polarimétrique parallèle $E_\parallel^F$ et la composante polarimétrique orthogonale $E_\perp^F$ du faisceau réfléchi sont dirigées respectivement vers le photodétecteur 28, et vers le photodétecteur 30. Les photodétecteurs 28, 30 délivrent chacun un photocourant, ci-après appelé signal électrique, aux moyens de calcul 9.

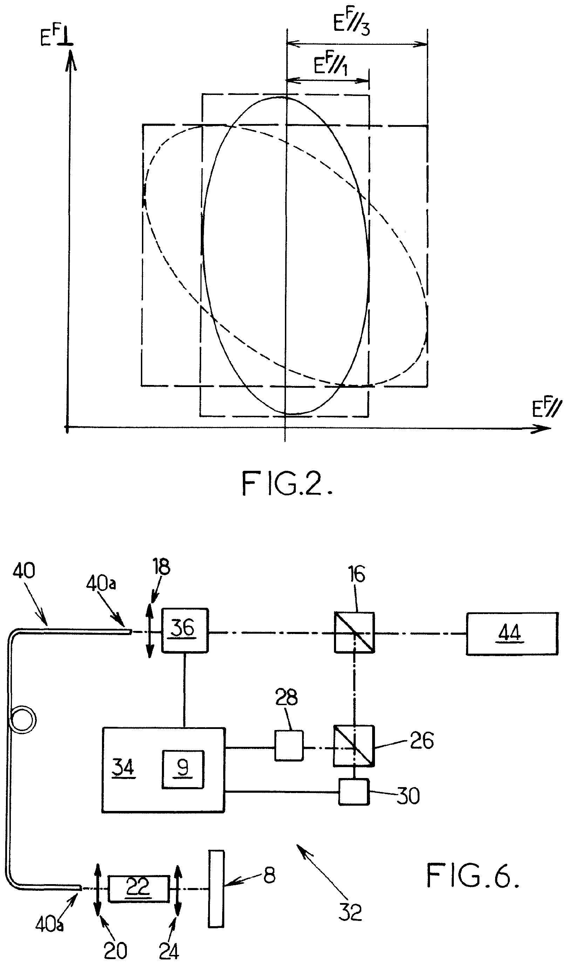

Les composantes polarimétriques $E_\parallel^F$ et $E_\perp^F$ varient au cours du temps en raison des variations de polarisation générées par les moyens de variation 19. Un exemple de variation de la composante polarimétrique parallèle $E_\parallel^F$ est représenté sur la figure 2 pour un échantillon cible présentant un déphasage de 90 degrés entre ses axes propres.

Selon l'invention, les moyens de calcul 9 sont propres à sélectionner la valeur maximale du signal électrique représentatif de la composante polarimétrique parallèle $E_\parallel^F$ ainsi que la valeur correspondante du signal électrique représentatif de la composante polarimétrique perpendiculaire $E_\perp^F$, c'est-à-dire la valeur mesurée au même moment ou en d'autres termes la valeur minimale de celui-ci.

Les moyens de calcul 9 sont aussi propres à sélectionner la valeur minimale du signal électrique représentatif de la composante polarimétrique parallèle $E_\parallel^F$ ainsi que la valeur correspondante du signal électrique représentatif de la composante polarimétrique perpendiculaire $E_\perp^F$, c'est-à-dire la valeur mesurée au même moment ou en d'autres termes la valeur maximale de celui-ci.

Dans l'exemple illustré sur la figure 2, la composante polarimétrique parallèle maximale est la composante polarimétrique $E_{\parallel 3}^F$.
Les moyens de calcul 9 sont propres à calculer le degré de polarisation (DOP) du faisceau en provenance du point de mesure de l'échantillon cible à partir de la formule suivante : $DOP = 1 - 2K$ où $K = P_{\parallel min}/(P_{\perp max} + P_{\parallel min})$
Dans laquelle :

- $P_{\parallel min}$ est la puissance minimale obtenue à partir de la valeur minimale du signal électrique sélectionné par les moyens de calcul 9. Elle est représentative de la composante polarimétrique parallèle $E_\parallel^F$ du champ électrique. $P_{\perp max}$ est la puissance représentative de la composante polarimétrique perpendiculaire $E_\perp^F$ du champ électrique mesurée au même moment.
- $(P_{\perp max} + P_{\parallel min})$ est la puissance totale récupérée par les photodétecteurs 28 et 30. Cette puissance est invariante d'une mesure à l'autre.

Ainsi, si $P_{\parallel min} = 0$ alors DOP=1 ce qui indique que l'échantillon cible est non dépolarisant ; si $P_{\parallel min} = P_{\perp max}$ alors DOP=0, ce qui indique que l'échantillon cible est totalement dépolarisant.

Les moyens de calcul 9 sont aussi propres à calculer le déphasage $\theta$ introduit entre les axes propres de l'échantillon cible à partir de la formule suivante :

$$\sin^2\left(\frac{\theta}{2}\right) = \frac{(1-K).P_{//max} - K.P_{\perp min}}{(1-2K)(P_{//max} + P_{\perp min})}$$

Dans laquelle :

- $P_{\parallel max}$ est la puissance maximale obtenue à partir de la valeur maximale du signal électrique sélectionné par les moyens de calcul 9. Elle est représentative de la composante polarimétrique parallèle $E_\parallel^F$ du champ électrique.

- $P_{\perp min}$ est la puissance représentative de la composante polarimétrique perpendiculaire $E_\perp^F$ du champ électrique mesurée au même moment.

En variante, les moyens de calcul 9 sont propres à sélectionner la valeur moyenne du signal électrique représentatif de la composante polarimétrique parallèle $E_{\parallel}^F$ ainsi que la valeur correspondante du signal électrique représentatif de la composante polarimétrique perpendiculaire $E_{\perp}^F$ mesurée au même moment. Dans ce cas, les moyens de calcul 9 sont propres à calculer le déphasage associé au point de mesure de l'échantillon cible lorsque celui-ci n'est pas dépolarisant, à partir de la formule suivante :

$$\theta = f(x) \text{ avec } x = P_{\parallel\,moyen} / (P_{\perp moyen} + P_{\parallel\,moyen})$$

Dans laquelle :

- f est une fonction continûment croissante entre 0 et 180° lorsque x varie de 0 à 1.

- $P_{\parallel\,moyen}$ est la puissance moyenne obtenue à partir de la moyenne de plusieurs mesures du signal électrique représentatif de la composante polarimétrique parallèle $E_{\parallel}^F$ associées à un point donné de la cible.

- $(P_{\perp moyen} + P_{\parallel moyen})$ est la puissance moyenne récupérée par les photodétecteurs 28 et 30 pendant une plage de temps prédéfinie.

La figure 3 représente un imageur polarimétrique 32 selon un premier mode de réalisation de l'invention. L'imageur 32 est formé à partir d'un dispositif 2 de détermination d'une information de polarisation, tel que décrit ci dessus, équipé d'une unité 34 de construction d'une image polarimétrique et d'un système de balayage 36.

L'unité de construction 34 est propre à recevoir les valeurs du degré de polarisation du faisceau et les valeurs du déphasage provenant de plusieurs points de mesure de l'échantillon cible 8 et à construire deux images polarimétriques à partir de celles-ci. Chaque niveau de gris ou chaque chrominance d'un pixel de la première image représente le degré de polarisation associé à un point de mesure de l'échantillon cible. Chaque niveau de gris ou chaque chrominance d'un pixel de la deuxième image représente le déphasage à un point de mesure de l'échantillon cible.

A cet effet, l'unité de construction d'image 34 est synchronisée au système de balayage 36.

Le système de balayage 36 est propre à diriger le faisceau incident vers plusieurs points de mesure de l'échantillon cible 8.

Il est disposé en aval du guide d'onde 6 en considérant le sens de parcours du faisceau incident. En particulier, il est disposé entre l'extrémité distale 6b du guide d'onde et le premier système optique 20.

Il est par exemple constitué par deux miroirs oscillants, l'un selon un axe vertical, et l'autre selon un axe horizontal, à une fréquence correspondant à la fréquence de construction d'une image par l'unité 34. Il est connecté à l'unité de construction 34 par un fil électrique 38.

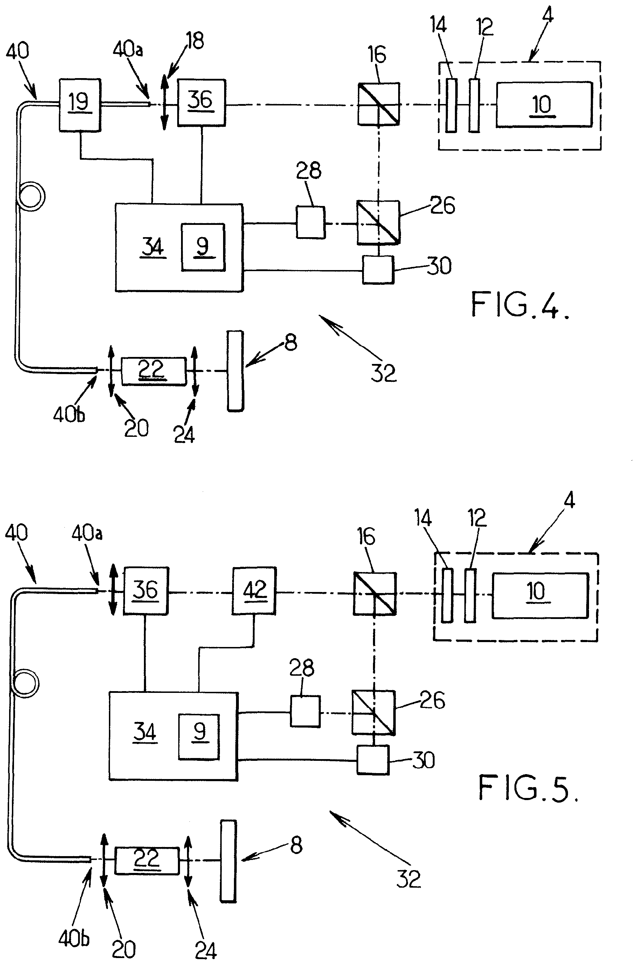

La figure 4 représente un imageur polarimétrique 32 selon un second mode de réalisation de l'invention. Il est similaire à l'imageur représenté sur la figure 3.

Toutefois, le guide d'onde 6 est remplacé par plusieurs guides d'onde 40, ou par une fibre optique multicœur, et le système de balayage 36 est disposé en amont des guides d'onde en considérant le sens du faisceau incident. Le système de balayage 36 est propre à diriger le faisceau incident tour à tour vers chaque guide d'onde de sorte que le faisceau éclaire successivement plusieurs points de mesure de l'échantillon cible 8. A la réception, le système de balayage traite séquentiellement le faisceau réfléchi. L'unité de construction 34 est synchronisée avec le système de balayage 36 de manière à pouvoir attribuer à chaque information de polarisation calculée par l'unité de calcul 9 une position correspondante sur l'échantillon cible 8.

La figure 5 représente un imageur polarimétrique 32 non couvert par les revendications. Il est similaire à l'imageur représenté sur la figure 4. Toutefois, les moyens 19 de variation de la biréfringence sont remplacés par des moyens 42 de variation de la polarisation montés entre le cube 16 et le système de balayage 36.

Les moyens 42 de variation de la polarisation sont constitués, par exemple, par un brouilleur de polarisation ou un agencement de lames de phase commandées par l'unité de calcul 9.

Les moyens 42 de variation de la polarisation peuvent également être utilisés (à la place des moyens 19 de variation de la biréfringence) dans le dispositif de détermination illustré sur la figure 1, ainsi que dans les imageurs illustrés sur les figures 2 à 4.

La figure 6 représente un imageur polarimétrique 32 selon un quatrième mode de réalisation de l'invention. Il est similaire à l'imageur représenté sur la figure 5. Toutefois, la source lumineuse monochromatique a été remplacée par une source polychromatique 44 et les moyens de variation de la polarisation 42 ont été supprimés. La source polychromatique est, par exemple, constituée par une diode superluminescente. Comme la biréfringence des guides d'onde varie en fonction de la longueur d'onde du faisceau les traversant, la variation de polarisation réalisée précédemment par les moyens de variation 19 ou 42, est ici induite par le passage dans les guides d'onde d'un faisceau à spectre élargi.

Dans ce cas, les moyens de calcul 9 sont propres à sélectionner la valeur maximale et la valeur minimale du signal électrique représentatif de la composante polarimétrique parallèle $E_{\parallel}^F$ parmi les composantes du champ électrique ayant des longueurs d'onde différentes. La valeur de la composante polarimétrique perpendiculaire $E_{\perp}^F$ ayant la même longueur d'onde est sélectionnée pour calculer le degré de polarisation et le déphasage.

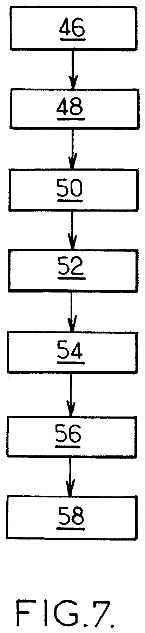

L'invention concerne également un procédé de détermination d'une information de polarisation illustré sur la figure 7. Le procédé débute par une étape 46 d'émission d'un faisceau lumineux incident polarisé rectilignement.

A cours d'une étape 48, le faisceau incident est guidé vers le point de mesure de l'échantillon cible à l'aide du guide d'onde 6.

Au cours d'une étape 50, deux composantes polarimétriques orthogonales du faisceau incident sont tournées d'un angle de 45 degrés par le rotateur de Faraday 22.

Au cours d'une étape 52, le faisceau incident est rétro-réfléchi sur le point de mesure de l'échantillon cible.

Au cours d'une étape 54, deux composantes polarimétriques orthogonales du faisceau réfléchi sont tournées d'un angle de 45 degrés. Puis, le faisceau réfléchi est injecté dans le guide d'onde 6 par le système optique 20.

Au cours d'une étape 56, le faisceau réfléchi est guidé vers l'unité de calcul 9 par le même guide d'onde 6.

Enfin, au cours d'une étape 58, l'information de polarisation du point de mesure de l'échantillon cible est calculé à partir du faisceau réfléchi récupéré à la sortie du guide d'onde 6.

Le déphasage permet d'obtenir, par exemple, des informations sur la biréfringence de l'échantillon cible tandis que le degré de polarisation permet d'obtenir des informations sur sa capacité à dépolariser la lumière.

En variante, le dispositif de détermination 2 où l'imageur polarimétrique 32 comprend un premier rotateur disposé uniquement sur le trajet du faisceau incident entre la partie distale 6b et l'échantillon cible 8, et un second rotateur disposé uniquement sur le trajet du faisceau réfléchi entre la partie distale 6b et l'échantillon cible 8. Le premier rotateur est propre à faire tourner les composantes polarimétriques du faisceau d'un angle $\alpha$. Le second rotateur est propre à faire tourner les composantes polarimétriques d'un angle 90- $\alpha$ degrés ; $\alpha$ étant compris entre 0 et 90 degrés.

Dans ce cas, le faisceau incident n'est pas perpendiculaire à la surface de l'échantillon cible.

## Revendications

1. Dispositif (2) de détermination d'au moins une information de polarisation d'un point de mesure d'un échantillon cible (8), le dispositif (2) comportant :

- une source lumineuse (4 ; 44) propre à émettre un faisceau lumineux polarisé rectilignement selon une direction prédéfinie, le faisceau lumineux étant destiné à être réfléchi par le point de mesure de l'échantillon cible (8) ;
- des moyens de détection (28, 30) et de calcul (9) de l'information de polarisation du point de mesure à partir du faisceau réfléchi par l'échantillon cible (8) ;
- au moins un guide d'onde (6 ; 40) apte à guider le faisceau incident vers l'échantillon cible (8), et le faisceau réfléchi vers les moyens (9) de calcul ; le guide d'onde (6 ; 40) étant une fibre optique monomode à la ou à chaque longueur d'onde du faisceau émis par la source lumineuse (4 ; 44) **caractérisé en ce qu'**il comprend en outre :
- des moyens (22) de rotation de la polarisation comprenant un rotateur de Faraday, propres à faire tourner deux composantes polarimétriques orthogonales $(E_{||}^{I}, E_{\perp}^{I})$ du faisceau incident après passage dans le guide d'onde (6 ; 40) et deux composantes polarimétriques $(E_{||}^{R}, E_{\perp}^{R})$ orthogonales du faisceau réfléchi avant passage dans le guide d'onde (6 ; 40) de manière à compenser l'effet de la biréfringence du guide d'onde (6; 40) ; et
- des moyens (19 ; 44) de variation de la polarisation du faisceau incident et du faisceau réfléchi, qui sont soit sous la forme de moyens (19) de variation de la biréfringence du guide d'onde montés sur le guide d'onde, pour lesquels la source de lumière (4) est monochromatique et qui sont adaptés pour modifier au cours du temps et de façon identique les composantes polarimétriques orthogonales $(E_{||}^{I}, E_{\perp}^{I})$ du faisceau incident après passage dans le guide d'onde et les composantes orthogonales $(E_{||}^{F}, E_{\perp}^{F})$ de la polarisation du faisceau réfléchi après passage dans le guide d'onde, soit qui sont fournis par la source de lumière sous forme d'une source de lumière polychromatique (44), la dépendance chromatique de la biréfringence du guide d'onde formant alors lesdits moyens de variation de la polarisation du faisceau incident et du faisceau réfléchi, les moyens (19 ; 44) de variation de la polarisation du faisceau incident et du faisceau réfléchi étant tels qu'ils permettent de calculer l'information de polarisation du point de mesure à partir des mesures pour chaque polarisation du faisceau incident.

2. Dispositif (2) selon la revendication 1, dans lequel les moyens de rotation (22) sont propres à faire tourner lesdites composantes polarimétriques $(E_{||}^{I} ; E_{\perp}^{I})$ du faisceau incident et lesdites composantes polarimétriques $(E_{||}^{R} ; E_{\perp}^{R})$ du faisceau réfléchi, dans un même sens de rotation.

**3.** Dispositif (2) selon l'une quelconque des revendications 1 et 2, dans lequel les moyens de rotation (22) comprennent un unique rotateur de Faraday propre à faire tourner lesdites composantes polarimétriques ($E_\parallel^I$ ; $E_\perp^I$) du faisceau incident et lesdites composantes polarimétriques ($E_\parallel^R$ ; $E_\perp^R$) du faisceau réfléchi d'un angle de 45 degrés.

**4.** Dispositif (2) selon l'une quelconque des revendications 1 et 2, dans lequel les moyens de rotation (22) comprennent au moins deux rotateurs de Faraday propres à faire tourner lesdites composantes polarimétriques ($E_\parallel^I$ ; $E_\perp^I$) du faisceau incident et lesdites composantes polarimétriques ($E_\parallel^R$ ; $E_\perp^R$) du faisceau réfléchi, l'un d'un angle de $\alpha$ degrés, l'autre d'un angle de 90- $\alpha$ degrés ; $\alpha$ étant un nombre compris entre 0 et 90 degrés.

**5.** Dispositif (2) selon l'une quelconque des revendications 1 à 4, dans lequel le guide d'onde (6 ; 40) comporte une extrémité proximale (6a ; 40a) destinée à être disposée du côté de la source lumineuse (4 ; 44), et une extrémité distale (6b ; 40b) destinée à être disposée du côté de l'échantillon cible (8), les moyens de rotation (22) étant disposés entre l'échantillon cible (8) et l'extrémité distale (6b ; 40b) du guide d'onde (6 ; 40).

**6.** Dispositif (2) selon l'une quelconque des revendications 1 à 5, qui comporte :

- des moyens (26, 28, 30) de mesure du faisceau réfléchi aptes à délivrer un signal électrique représentatif de la composante polarimétrique du faisceau réfléchi ($E_\parallel^{RR}$) orientée selon la direction prédéfinie, appelé signal parallèle, et un signal électrique représentatif de la composante polarimétrique perpendiculaire ($E_\perp^{RR}$) à celle-ci, appelé signal orthogonal ; et dans lequel les moyens de calcul (9) comportent :
- des moyens de sélection d'une valeur du signal parallèle, le signal parallèle variant au cours du temps ou en fonction de la longueur d'onde ; et
- des moyens de mesure de l'information de polarisation du point de mesure de l'échantillon cible (8) à partir du signal parallèle sélectionné et du signal orthogonal mesuré au même moment ou du signal orthogonal présentant la même longueur d'onde.

**7.** Dispositif (2) selon la revendication 6, dans lequel la valeur du signal parallèle sélectionnée est la valeur minimale de celui-ci, ou la valeur maximale de celui-ci ou la valeur moyenne de celui-ci.

**8.** Imageur polarimétrique (32) propre à générer une image polarimétrique d'un échantillon cible (8), l'imageur comportant :

- un dispositif (2) de détermination d'une information de polarisation selon l'une quelconque des revendications 1 à 7, ledit dispositif (2) étant propre à déterminer plusieurs informations de polarisation ;
- une unité (34) de construction d'une image polarimétrique représentative des informations de polarisation des points de mesure de l'échantillon cible (8), chaque caractéristique d'un pixel de l'image représentant l'information de polarisation d'un point de mesure de l'échantillon cible (8).

**9.** Imageur polarimétrique (32) selon la revendication 8, qui comporte plusieurs guides d'onde (40) et un système de balayage (36) disposé en amont desdits guides d'onde (40) en considérant le sens du faisceau incident, le système de balayage (36) étant propre à diriger le faisceau incident vers plusieurs points de mesure de l'échantillon cible (8), le système de balayage (36) étant commandé par l'unité de construction d'image (34) et étant synchronisé à celle-ci.

**10.** Imageur polarimétrique selon la revendication 8, qui comporte un unique guide d'onde (6) et un système de balayage (36) disposé en aval du guide d'onde (6) en considérant le sens du faisceau incident, le système de balayage (36) étant propre à diriger le faisceau incident vers plusieurs points de mesure de l'échantillon cible (8), le système de balayage (36) étant commandé par l'unité de construction d'image (34) et étant synchronisé à celle-ci.

**11.** Procédé de détermination d'au moins une information sur la polarisation en un point de mesure d'un échantillon cible (8), le procédé comportant les étapes suivantes :

- émission (46) d'un faisceau lumineux incident polarisé rectilignement selon une direction prédéfinie ;
- guidage (48) du faisceau incident vers le point de mesure de l'échantillon cible (8) à l'aide d'un guide d'onde (6 ; 40), le guide (6 ; 40) étant une fibre monomode à la ou à chaque longueur d'onde du faisceau incident ;
- rotation (50) de deux composantes polarimétriques orthogonales du faisceau incident après passage dans le guide d'onde (6 ; 40) pour compenser l'effet de la biréfringence du guide d'onde (6, 40) avec des moyens (22) de rotation de la polarisation comprenant un rotateur de Faraday ;
- réflexion (52) du faisceau incident sur le point de mesure de l'échantillon cible (8) ;
- rotation (54) de deux composantes polarimétriques orthogonales du faisceau réfléchi avant passage dans le guide d'onde (6 ; 40) pour compenser l'effet de la biréfringence du guide d'onde (6 ; 40) avec lesdits moyens (22) de rotation de la polarisation ;

- guidage (56) du faisceau réfléchi vers une unité de calcul (9) par le même guide d'onde (6 ; 40) ;
- variation de la de la polarisation du faisceau incident et du faisceau réfléchi avec des moyens de variation de la polarisation du faisceau incident et du faisceau réfléchi, qui sont soit sous la forme de moyens (19) de variation de la biréfringence du guide d'onde montés sur le guide guide d'onde pour lesquels la source de lumière (4) est monochromatique et qui sont adaptés pour modifier au cours du temps et de façon identique les composantes polarimétriques orthogonales $(E_{\parallel}^{I}, E_{\perp}^{I})$ du faisceau incident après passage dans le guide d'onde et les composantes orthogonales $(E_{\parallel}^{F}, E_{\perp}^{F})$ de la polarisation du faisceau réfléchi après passage dans le guide d'onde, soit qui sont fournis par la source de lumière sous forme d'une source de lumière polychromatique (44), la dépendance chromatique de la biréfringence du guide d'onde formant alors les moyens de variation de la polarisation du faisceau incident et du faisceau réfléchi ;

- détection du faisceau réfléchi pour chaque polarisation du faisceau incident et calcul (58) de l'information de polarisation du point de mesure de l'échantillon cible (8) à partir du faisceau réfléchi récupéré à la sortie du guide d'onde (6 ; 40), à partir des mesures obtenues pour chaque polarisation du faisceau incident.

**Patentansprüche**

1. Vorrichtung (2) zum Bestimmen mindestens einer Polarisationsinformation eines Messpunkts einer Zielprobe (8), wobei die Vorrichtung (2) Folgendes aufweist:

   - eine Lichtquelle (4; 44), welche in der Lage ist, einen polarisierten Lichtstrahl geradlinig in eine vorbestimmte Richtung auszusenden, wobei der Lichtstrahl dazu bestimmt ist, durch den Messpunkt der Zielprobe (8) reflektiert zu werden;
   - Mittel zum Erfassen (28, 30) und Berechnen (9) der Polarisationsinformation des Messpunkts anhand des durch die Zielprobe (8) reflektierten Strahls;
   - mindestens einen Hohlwellenleiter (6; 40), welcher in der Lage ist, den einfallenden Strahl in Richtung der Zielprobe (8), und den reflektierten Strahl in Richtung der Berechnungsmittel (9) zu führen; wobei der Hohlwellenleiter (6; 40) ein Monomode-Lichtwellenleiter bei der oder bei jeder Wellenlänge des durch die Lichtquelle (4; 44) ausgesendeten Strahls ist, **dadurch gekennzeichnet, dass** er ferner Folgendes umfasst:

   - Mittel (22) zur Drehung der Polarisation, umfassend einen Faraday-Rotator, welche in der Lage sind, zwei polarimetrische rechtwinklige Komponenten ($E_{\parallel}^{I}$, $E_{\perp}^{I}$) des einfallenden Strahls nach Passieren durch den Hohlwellenleiter (6; 40) und zwei polarimetrische rechtwinklige Komponenten ($E_{\parallel}^{R}$, $E_{\perp}^{R}$) des reflektierten Strahls vor Passieren durch den Hohlwellenleiter (6; 40) in einer Weise zu drehen, dass der Effekt der Doppeltbrechung des Hohlwellenleiters (6; 40) kompensiert wird; und
   - Mittel (19; 44) zur Variation der Polarisation des einfallenden Strahls und des reflektierten Strahls, welche entweder in Form von Mitteln (19) zur Variation der Doppeltbrechung des Hohlwellenleiters vorliegen, welche an dem Hohlwellenleiter montiert sind, für welche die Lichtquelle (4) monochromatisch ist und die geeignet sind, im Zeitverlauf und in identischer Weise die rechtwinkligen polarimetrischen Komponenten ($E_{\parallel}^{I}$, $E_{\perp}^{I}$) des einfallenden Strahls nach Passieren durch den Hohlwellenleiter und die rechtwinkligen Komponenten ($E_{\parallel}^{F}$, $E_{\perp}^{F}$) der Polarisation des reflektierten Strahls nach Passieren durch den Hohlwellenleiter zu modifizieren, oder welche durch die Lichtquelle in Form einer polychromatischen Lichtquelle (44) bereitgestellt werden, wobei die chromatische Abhängigkeit der Doppeltbrechung des Hohlwellenleiters so die Mittel zur Variation der Polarisation des einfallenden Strahls und des reflektierten Strahls bildet, wobei die Mittel (19; 44) zur Variation der Polarisation des einfallenden Strahls und des reflektierten Strahls so geartet sind, dass sie es ermöglichen, die Polarisationsinformation des Messpunkts anhand der Messungen für jede Polarisation des einfallenden Strahls zu berechnen.

2. Vorrichtung (2) nach Anspruch 1, wobei die Drehmittel (22) in der Lage sind, die polarimetrischen Komponenten ($E_{\parallel}^{I}$, $E_{\perp}^{I}$) des einfallenden Strahls und die polarimetrischen Komponenten ($E_{\parallel}^{R}$, $E_{\perp}^{R}$) des reflektierten Strahls in eine gleiche Drehrichtung zu drehen.

3. Vorrichtung (2) nach einem der Ansprüche 1 und 2, wobei die Drehmittel (22) einen einzigen Faraday-Rotator umfassen, welcher in der Lage ist, die polarimetrischen Komponenten ($E_{\parallel}^{I}$, $E_{\perp}^{I}$) des einfallenden Strahls und die polarimetrischen Komponenten

($E^R_\parallel$, $E^R_\perp$) des reflektierten Strahls um einen Winkel von 45 Grad zu drehen.

4. Vorrichtung (2) nach einem der Ansprüche 1 und 2, wobei die Drehmittel (22) mindestens zwei Faraday-Rotaroren umfassen, welche in der Lage sind, die polarimetrischen Komponenten ($E^I_\parallel$, $E^I_\perp$) des einfallenden Strahls und die polarimetrischen Komponenten ($E^R_\parallel$, $E^R_\perp$) des reflektierten Strahls, eines um einen Winkel von $\alpha$ Grad und das andere um einen Winkel von 90- $\alpha$ Grad zu drehen; wobei $\alpha$ eine Zahl zwischen 0 und 90 Grad ist.

5. Vorrichtung (2) nach einem der Ansprüche 1 bis 4, wobei der Hohlwellenleiter (6; 40) ein proximales Ende (6a; 40a) aufweist, welches dazu bestimmt ist, auf der Seite der Lichtquelle (4; 44) angeordnet zu sein, und ein distales Ende (6b; 40b), welches dazu bestimmt ist, auf der Seite der Zielprobe (8) angeordnet zu sein, wobei die Drehmittel (22) zwischen der Zielprobe (8) und dem distalen Ende (6b; 40b) des Hohlwellenleiters (6; 40) angeordnet sind.

6. Vorrichtung (2) nach einem der Ansprüche 1 bis 5, Folgendes aufweist:

   - Mittel (26, 28, 30) zum Messen des reflektierten Strahls, welche in der Lage sind, ein elektrisches Signal abzugeben, welches die polarimetrische Komponente des reflektierten Strahls ($E^{RR}_\parallel$) darstellt, welche entlang der vorbestimmten Richtung ausgerichtet ist, genannt paralleles Signal, und ein elektrisches Signal, welches die polarimetrische Komponente ($E^{RR}_\perp$) darstellt, zu dieser rechtwinklig ist, genannt rechtwinkliges Signal; und wobei die Berechnungsmittel (9) Folgendes aufweisen:
   - Mittel zur Auswahl eines Wertes des parallelen Signals, wobei das parallele Signal im Zeitverlauf oder in Abhängigkeit von der Wellenlänge variiert; und
   - Mittel zur Messung der Polarisationsinformation des Messpunkts der Zielprobe (8) anhand des gewählten parallelen Signals und des zum gleichen Zeitpunkt gemessenen rechtwinkligen Signals, oder des rechtwinkligen Signals, welches dieselbe Wellenlänge aufweist.

7. Vorrichtung (2) nach Anspruch 6, wobei der Wert des gewählten parallelen Signals der Mindestwert desselben, oder der maximale Wert desselben oder der Mittelwert desselben ist.

8. Polarimetrische Bildgebungsvorrichtung (32), welche in der Lage ist, ein polarimetrisches Bild einer Zielprobe (8) zu erzeugen, wobei die Bildgebungsvorrichtung Folgendes umfasst:

   - eine Vorrichtung (2) zum Bestimmen einer Polarisationsinformation nach einem der Ansprüche 1 bis 7, wobei die Vorrichtung (2) geeignet ist, mehrere Polarisationsinformationen zu bestimmen;
   - eine Einheit (34) zur Konstruktion eines polarimetrischen Bildes, welches Polarisationsinformationen der Messpunkte der Zielprobe (8) darstellt, wobei jedes Merkmal eines Pixels des Bildes die Polarisationsinformation eines Messpunkts der Zielprobe (8) darstellt.

9. Polarimetrische Bildgebungsvorrichtung (32) nach Anspruch 8, welche mehrere Hohlwellenleiter (40) und ein Abtastsystem (36) aufweisen, welches im vorgelagerten Bereich der Hohlwellenleiter (40) unter Betrachtung der Richtung des einfallenden Strahls angeordnet ist, wobei das Abtastsystem (36) geeignet ist, den einfallenden Strahl in Richtung mehrerer Messpunkte der Zielprobe (8) zu richten, wobei das Abtastsystem (36) durch die Bildkonstruktionseinheit (34) gesteuert wird und mit dieser synchronisiert ist.

10. Polarimetrische Bildgebungsvorrichtung nach Anspruch 8, welche einen einzigen Hohlwellenleiter (6) und ein Abtastsystem (36) aufweist, welches im nachgelagerten Bereich des Hohlwellenleiters (6) unter Betrachtung der Richtung des einfallenden Strahls angeordnet ist, wobei das Abtastsystem (36) in der Lage ist, den einfallenden Strahl in Richtung mehrerer Messpunkte der Zielprobe (8) zu richten, wobei das Abtastsystem (36) durch die Bildkonstruktionseinheit (34) gesteuert wird und mit dieser synchronisiert ist.

11. Verfahren zur Bestimmung mindestens einer Polarisationsinformation in einem Messpunkt einer Zielprobe (8), wobei das Verfahren folgende Schritte aufweist:

   - geradliniges Aussenden (46) eines einfallenden polarisierten Lichtstrahls in eine vorbestimmte Richtung;
   - Führen (48) des einfallenden Strahls in Richtung des Messpunkts der Zielprobe (8) mithilfe eines Hohlwellenleiters (6; 40), wobei der Leiter (6; 40) eine Monomode-Faser bei der oder bei jeder Wellenlänge des einfallenden Strahls ist,
   - Drehen (50) zweier rechtwinkliger polarimetrischer Komponenten des einfallenden Strahls nach Passieren durch den Hohlwellenleiter (6; 40) zum Kompensieren des Effekts der Doppelbrechung des Hohlwellenleiters (6; 40) mit Mitteln (22) zum Drehen der Polarisation, welche einen Faraday-Rotator umfassen;
   - Reflektieren (52) des einfallenden Strahls auf den Messpunkt der Zielprobe (8);

- Drehen (54) zweier rechtwinkliger polarimetrischer Komponenten des reflektierten Strahls vor Passieren durch den Hohlwellenleiter (6; 40) zum Kompensieren des Effekts der Doppeltbrechung des Hohlwellenleiters (6; 40) mit den Mitteln (22) zum Drehen der Polarisation;

- Führen (56) des reflektierten Strahls in Richtung einer Berechnungseinheit (9) durch denselben Hohlwellenleiter (6; 40),

- Variieren der Polarisation des einfallenden Strahls und des reflektierten Strahls mit Mitteln zum Variieren der Polarisation des einfallenden Strahls und des reflektierten Strahls, welche entweder in Form von Mitteln (19) zum Variieren der Doppeltbrechung des Hohlwellenleiters vorliegen, welche an dem Hohlwellenleiter montiert sind, für welche die Lichtquelle (4) monochromatisch ist und die geeignet sind, im Zeitverlauf und in identischer Weise die rechtwinkligen polarimetrischen Komponenten ($E^I_\parallel$, $E^I_\perp$) des einfallenden Strahls nach Passieren durch den Hohlwellenleiter und die rechtwinkligen Komponenten ($E^F_\parallel$, $E^F_\perp$) der Polarisation des reflektierten Strahls nach Passieren durch den Hohlwellenleiter zu modifizieren, oder welche durch die Lichtquelle in Form einer polychromatischen Lichtquelle (44) bereitgestellt werden, wobei die chromatische Abhängigkeit der Doppeltbrechung des Hohlwellenleiters so die Mittel zur Variation der Polarisation des einfallenden Strahls und des reflektierten Strahls bildet;

- Detektieren des reflektierten Strahls für jede Polarisation des einfallenden Strahls und Berechnen (58) der Polarisationsinformation des Messpunktes der Zielprobe (8) anhand des reflektierten Strahls, welcher am Ausgang des Hohlwellenleiters (6; 40) abgefangen wird, anhand der durch jede Polarisation des einfallenden Strahls erzielten Messungen.

**Claims**

1. A device (2) for determining at least one piece of polarization information of a measurement point of a target sample (8), said device (2) having:

   - a light source (4; 44) able to emit a rectilinearly polarized light beam in a predefined direction, the light beam being intended to be reflected by the measurement point of the target sample (8);
   - means for detecting (28, 30) and calculating (9) the piece of polarization information of the measurement point from the beam reflected by the target sample (8);
   - at least one waveguide (6; 40) able to guide the incident beam towards the target sample (8) and the reflected beam towards the calculation means (9); the waveguide (6; 40) being a single-mode optical fiber at the or at each wavelength of the beam emitted by the light source (4; 44) **characterized in that** it further comprises:

   - means (22) of rotating the polarization comprising a Faraday rotator able to rotate two orthogonal polarimetric components ($E^I_\parallel$, $E^I_\perp$) of the incident beam after passage through the waveguide (6; 40) and two orthogonal polarimetric components ($E^R_\parallel$, $E^R_\perp$) of the reflected beam before passage through the waveguide (6; 40) to compensate for the effect of the birefringence of the waveguide (6; 40), and
   - means (19; 44) of varying the polarization of the incident beam and of the reflected beam, which are either in the form of means (19) for varying the birefringence of the waveguide, which are mounted on the waveguide, for which the light source (4) is monochromatic and which are adapted to modify, over time and in an identical manner, the orthogonal polarimetric components ($E^I_\parallel$, $E^I_\perp$) of the incident beam after passage through the waveguide and the orthogonal polarimetric components ($E^F_\parallel$, $E^F_\perp$) of the polarization of the reflected beam after passage through the waveguide, or are provided by the light source in the form of a polychromatic light source (44), the chromatic dependency of the birefringence of the waveguide thereby forming said means for varying the polarization of the incident beam and the reflected beam, the means (19; 44) for varying the polarization of the incident beam and of the reflected beam being such that they enable the calculation of the piece of polarization information for the measurement point from the measurements for each polarization of the incident beam.

2. The device (2) according to claim 1, wherein the rotation means (22) are able to rotate said polarimetric components ($E^I_\parallel$, $E^I_\perp$) of the incident beam and said polarimetric components ($E^R_\parallel$, $E^R_\perp$) of the reflected beam in the same direction of rotation.

3. The device (2) according to any of claims 1 and 2, wherein the rotation means (22) comprise a single Faraday rotator able to rotate said polarimetric components ($E^I_\parallel$, $E^I_\perp$) of the incident beam and said polarimetric components ($E^R_\parallel$, $E^R_\perp$) of the reflected beam by a 45 degree angle.

4. The device (2) according to any one of claims 1 and 2, wherein the rotation means (22) comprise at least two Faraday rotators able to rotate said polarimetric components ($E^I_\parallel$, $E^I_\perp$) of the incident beam and said polarimetric components ($E^R_\parallel$, $E^R_\perp$) of the reflected beam, one by an angle of $\alpha$ degrees, the other by an angle of $90-\alpha$ degrees, where $\alpha$ is a number be-

tween 0 and 90 degrees.

5. The device (2) according to any one of claims 1 to 4, wherein the waveguide (6; 40) has a proximal end (6a; 40a) intended to be placed on the side of the light source (4; 44), and a distal end (6b; 40b) intended to be placed on the side of the target sample (8), the rotation means (22) being placed between the target sample (8) and the distal end (6b; 40b) of the waveguide (6; 40).

6. The device (2) according to any one of claims 1 to 5, which has:

- means (26, 28, 30) for measuring the reflected beam, which are able to deliver an electric signal representative of the polarimetric component of the reflected beam ($E_\parallel^{RR}$) oriented in the predefined direction, called a parallel signal, and an electric signal representative of the polarimetric component ($E_\perp^{RR}$) perpendicular thereto, called an orthogonal signal; and wherein the calculation means (9) have:
- means for selecting a value of the parallel signal, the parallel signal varying over time or as a function of the wavelength; and
- means for measuring the piece of polarization information of the measurement point of the target sample (8) from the selected parallel signal and from the orthogonal signal measured at the same moment or from the orthogonal signal having the same wavelength.

7. The device (2) according to claim 6, wherein the selected value of the parallel signal is its minimum value, or its maximum value, or its mean value.

8. A polarimetric imaging device (32) able to generate a polarimetric image of a target sample (8), with said imaging device comprising:

- a device (2) for determining a piece of polarization information according to any one of claims 1 to 7, said device (2) being able to determine multiple pieces of polarization information;
- a unit (34) for constructing a polarimetric image representative of the polarization information for the measurement points of the target sample (8), each characteristic of a pixel of the image representing the piece of polarization information of a measurement point of the target sample (8).

9. The polarimetric imaging device (32) according to claim 8, having multiple waveguides (40) and a scanning system (36) placed upstream from said waveguides (40) when considering the direction of the incident beam; said scanning system (36) being able to direct the incident beam towards multiple measurement points of the target sample (8), said scanning system (36) being controlled by the image construction unit (34) and being synchronized with the latter.

10. The polarimetric imaging device according to claim 8, having a single waveguide (6) and a scanning system (36) placed downstream from the waveguide (6) when considering the direction of the incident beam; said scanning system (36) being able to direct the incident beam towards multiple measurement points of the target sample (8), said scanning system (36) being controlled by the image construction unit (34) and being synchronized with the latter.

11. A method for determining at least one piece of polarization information at a measurement point of a target sample (8), the method having the following steps:

- emitting (46) a rectilinearly polarized incident light beam in a predefined direction,
- guiding (48) the incident beam towards the measurement point of the target sample (8) with the aid of a waveguide (6; 40), the guide (6; 40) being a single-mode fiber at the or at each wavelength of the incident beam;
- rotating (50) two orthogonal polarimetric components of the incident beam after passage through the waveguide (6; 40), to compensate for the effect of the birefringence of the waveguide (6; 40) with polarization rotation means (22) comprising a Faraday rotator;
- reflecting (52) the incident beam on the measurement point of the target sample (8);
- rotating (54) two orthogonal polarimetric components of the reflected beam before passage through the waveguide (6; 40) to compensate for the effect of the birefringence of the waveguide (6; 40) with said polarization rotation means (22);
- guiding (56) the reflected beam towards a calculation unit (9) by the same waveguide (6; 40);
- varying the polarization of the incident beam and of the reflected beam with means for varying the polarization of the incident beam and of the reflected beam, which are either in the form of means (19) for varying the birefringence of the waveguide, which are mounted on the waveguide, for which the light source (4) is monochromatic and which are adapted to modify, over time and in an identical manner, the orthogonal polarimetric components ($E_\parallel^I$, $E_\perp^I$) of the incident beam after passage through the waveguide and the orthogonal components ($E_\parallel^F$, $E_\perp^F$) of the polarization of the reflected beam after passage through the waveguide, or are provided by the light source in the form of a polychromatic light source (44), the chromatic dependency of the birefringence of the waveguide thereby forming the means for varying the polarization of the incident beam and of the reflected beam;

- detecting the reflected beam for each polarization of the incident beam and calculating (58) the piece of polarization information of the measurement point of the target sample (8) from the reflected beam recovered at the output of the waveguide (6; 40), from the measurements obtained for each polarization of the incident beam.

FIG.1.

FIG.3.

FIG.2.

FIG.6.

FIG.4.

FIG.5.

FIG.7.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7289211 B

- US 6292287 B1

**Littérature non-brevet citée dans la description**

- **P. M. F. NIELSEN et al.** Polarization-sensitive scanned fiber confocal microscope. *Opt. Eng.,* Novembre 1996, vol. 35 (11), 3084-3091